# EUROPÄISCHE PATENTSCHRIFT

(11) **EP 1 693 078 B1**
(45) Veröffentlichungstag und Bekanntmachung des Hinweises auf die Patenterteilung: **01.08.2007**
(21) Anmeldenummer: 05003609.4
(22) Anmeldetag: 19.02.2005
(51) Int. Cl.: A61M 5/315, A61M 5/20, A61M 5/30

(54) **Verabreichungsvorrichtung**
Delivery device
Dispositif d'administration

(43) Veröffentlichungstag der Anmeldung: 23.08.2006
(73) Patentinhaber: TecPharma Licensing AG, 3401 Burgdorf (CH)
(72) Erfinder: Neracher, Arnold, 1247 Anières (CH)

(56) Entgegenhaltungen:
- WO-A-20/04006999
- DE-C- 856 793
- GB-A- 915 262
- GB-A- 1 274 917
- US-A- 4 214 584

## Beschreibung

Die vorliegende Erfindung betrifft eine Verabreichungsvorrichtung zur Verabreichung eines injizierbaren Produktes, insbesondere einen Autoinjektor, besonders ein nadelloses Injektionsgerät.

Verabreichungsvorrichtungen, mit denen ein injizierbares Produkt, wie beispielsweise Insulin oder Wachstumshormone, automatisch verabreicht werden kann, sind im Stand der Technik in verschiedenen Ausführungsformen bekannt. Diese sind so ausgelegt, dass die Verabreichung mit nur einem Handgriff oder Knopfdruck erfolgen kann. Grob können zwei verschiedene Typen an Autoinjektionsgeräten unterschieden werden. Der erste Typ weist eine Einstech- und Ausschütteinrichtung auf. Mit der Einsticheinrichtung wird eine Injektionsnadel in ein Körpergewebe eingestochen. Nach dem Einstechen der Injektionsnadel schüttet die Ausschütteinrichtung das zu injizierende Produkt aus. Bei dem zweiten Typ handelt es um einen nadellosen Autoinjektor. Hierbei erzeugt eine Ausschütteinrichtung beim Ausschüttvorgang einen so großen Druck, dass das zu injizierende Produkt die Haut penetriert, so dass das Produkt dem Körpergewebe verabreicht werden kann. Die vorliegende Erfindung bezieht sich auf beide Typen an Autoinjektoren. Sie ist universell bei Verabreichungsvorrichtungen aller Art einsetzbar.

Die Ausschütteinrichtung weist in der Regel einen Kolben auf, der in einem Gehäuse der Injektionsvorrichtung in eine Vortriebsrichtung bewegbar aufgenommen ist. Die Bewegung des Kolbens in Vortriebsrichtung wird über ein Antriebsmittel mit einem Druckmittel bewirkt. Solche Druckmittel können beispielsweise Torsionsfedern oder entropische Federn sein. Die Druckmittel sind in geladenem Zustand, d.h. vor dem Betätigen eines Auslösers, zwischen der Ausschütteinrichtung und einem Widerlager, beispielsweise am Gehäuse der Injektionsvorrichtung, eingespannt. Das Druckmittel ist folglich geladen. Um ein unbeabsichtigtes Auslösen zu verhindern, ist die Ausschütteinrichtung im gespannten Zustand fixiert. Durch Betätigung des Auslösers wird die Ausschütteinrichtung gelöst; der Ausschüttvorgang kann stattfinden. Hierbei bewegt sich aufgrund der Antriebskraft des gespannten Druckmittels die Ausschütteinrichtung, beispielsweise der Kolben, in Vortriebsrichtung und schüttet so das Produkt aus. Wie oben ausgeführt, kann dem Ausschüttvorgang bei bestimmten Injektionsgerättypen ein Einstechvorgang vorangehen.

Die Fixierung des Injektionsgerätes im geladenen Zustand erfolgt beispielsweise, wie aus der GB 1274917 bekannt, mit Hilfe von zwei Kugeln, die in der Ausgangsposition in einer Ausnehmung der Ausschütteinrichtung, genauer der Kolbenstange, arretiert sind. Durch die Betätigung des Auslösers wird die Arretierung gelöst, wobei die Kugeln frei werden und, bedingt durch die Vorspannung des Druckmittels, aus der Ausnehmung der Kolbenstange herausgedrängt werden. Die Kolbenstange ist nun frei beweglich und kann durch das Druckmittel in Vortriebsrichtung bewegt werden, wodurch der Kolben ebenfalls in Vortriebsrichtung bewegt wird. Damit kann der Ausschüttvorgang stattfinden.

Aus anderen im Stand der Technik bekannten Injektionsvorrichtungen, die demselben Prinzip folgen, ist bekannt, dass die Kugeln im gespannten Zustand sowohl die Einstech- als auch die Ausschütteinrichtung fixieren. Durch Freigabe der Kugeln werden diese Einrichtungen frei oder es wird beispielsweise zuerst nur die Einstecheinrichtung freigegeben, die zu einem späteren Zeitpunkt dann die Ausschütteinrichtung wieder freigibt. Es ist auch bekannt, anstelle von Kugeln kantenförmige Segmente vorzusehen, die mit einer Nute an der Kolbenstange zusammenwirken.

Ein Nachteil der bekannten Halteelemente, wie beispielsweise Kugeln oder kantenförmige Segmente, ist, dass die Kontaktoberfläche zwischen den Halteelementen und der Nute auf der Kolbenstange vergleichsweise gering ist. Gerade bei Kugeln liegt nur ein sehr geringer Prozentsatz der Kugeloberfläche überhaupt an der Kolbenstange an. Dadurch sind diese Vorrichtungen zur Freisetzung von großen Kräften nicht nutzbar. Die auf Kugeln basierenden Systeme sind aufgrund des Rollkoeffizienten, der notwendig ist, damit die Kugel auf der Oberfläche bei der Entlassbewegung rollen bzw. rutschen kann, nur aus gehärtetem Stahl realisierbar. Wollte man das Kugelsystem in mit Glas- oder Kohlenstofffaser armiertem Plastik realisieren, so wäre es aufgrund der sehr kleinen Fläche am Kontaktpunkt nur schwer möglich, den Auslöser zu betätigen. Zusammenfassend bedarf es folglich bei den gegenwärtig bekannten Halteelementen von Verabreichungsgeräten vergleichsweise großer Kräfte, um den Auslöser zu betätigen und damit das Antriebsmittel, zum Beispiel die Kolbenstange, freizugeben.

Aufgabe der vorliegenden Erfindung ist daher, eine Verabreichungsvorrichtung bereitzustellen, bei der das Auslösen der Verabreichung, insbesondere der Injektion, auf einfache Weise und gut funktionierend realisiert ist, wobei eine vergleichsweise geringe Auslösekraft aufgewendet werden soll.

Diese Aufgabe wird durch eine Verabreichungsvorrichtung nach Anspruch 1 gelöst. Vorteilhafte und weiterbildende Ausgestaltungen ergeben sich aus den Unteransprüchen.

Die erfindungsgemäße Verabreichungsvorrichtung umfasst ein Gehäuse, in dem ein zu applizierendes, insbesondere zu injizierendes Produkt aufgenommen ist. In der Regel befindet sich dieses in einer Ampulle, die vor der Verwendung in das Gehäuse eingesetzt wird. In dem Gehäuse ist weiter ein Kolben, auch oft als Stempel bezeichnet, bewegbar aufgenommen. Dieser Kolben ist in Vortriebsrichtung, d.h. in Richtung zum Auslass der Verabreichungsvorrichtung bewegbar, wodurch das Produkt ausgeschüttet wird. Weiter ist ein in Vortriebsrichtung auf ein Antriebsmittel, insbesondere eine Kolbenstange, des Kolbens wirkendes Druckmittel vorgesehen. Das Druckmittel kann beispielsweise eine Torsionsfeder oder eine entropische Feder sein. Das Antriebsmittel, beispielsweise die Kolbenstange, ist mit Hilfe eines Halteelementes gegen die Kraft des Druckmittels in einer Halteposition in einem Halteeingriff lösbar gehalten. Das Druckmittel ist somit in der Halteposition geladen, insbesondere gespannt. Das Halteelement muss folglich die Kraft des Druckmittels überwinden, um das Antriebsmittel in der Halteposition zu fixieren. Erfindungsgemäß ist das Halteelement ein zu einem umlaufenden Ring verschließbares Element. Dieses hält im geschlossenen Zustand das Antriebsmittel in Halteeingriff. Dabei ist das Halteelement vorgespannt. Im geöffneten Zustand gibt das Halteelement das Antriebsmittel frei, wodurch diese unter der Krafteinwirkung des Druckmittels in Vortriebsrichtung bewegt wird. Durch den Auslösevorgang wird folglich das Halteelement vom geschlossenen Zustand zum geöffneten Zustand verbracht. Da das Halteelement in Halteposition vorgespannt ist, ist es bestrebt, vom geschlossenen in den geöffneten Zustand überzugehen, so dass eine vergleichsweise kleine Auslösekraft vonnöten ist. Ein Vorteil liegt also darin, dass eine sehr große mechanische Kraft durch eine sehr schwache Belastungskraft freigesetzt wird.

Ein weiterer Vorteil des erfindungsgemäßen Halteelementes liegt darin, dass es im Wesentlichen an der gesamten Umfangsfläche des Antriebsmittels, beispielsweise der Kolbenstange anliegen kann; im Gegensatz zu den bekannten Haltemitteln wie Kugeln, welche nur punktförmig an der Kolbenstange anliegen. Der Auflagedruck verteilt sich bei der erfindungsgemäßen Lösung auf die gesamte Ringauflagefläche des Halteelementes. Im Gegensatz dazu liegt der gesamte Auflagedruck bei Kugeln auf vergleichsweise kleinen Punkten. Zur Verdeutlichung, die Kontaktfläche des erfindungsgemäßen Halteelementes mit einem Durchmesser von ca. 10 mm ist dabei ungefähr 1000-mal größer als die Kontaktfläche von zwei Kugeln mit jeweils einem Durchmesser von ca. 5 mm. Durch die große Kontaktfläche und damit die günstige Verteilung der Reibkraft auf eine große Fläche ist es möglich, alle Elemente der Verabreichungsvorrichtung aus Kunststoff zu fertigen, wodurch die Herstellungskosten reduziert werden. Das Halteelement ist bevorzugt aus Stahl, insbesondere Federstahl, Fiberglas oder Kohlenstofffasern gefertigt oder ist mit Faserverbundstoffen armiert.

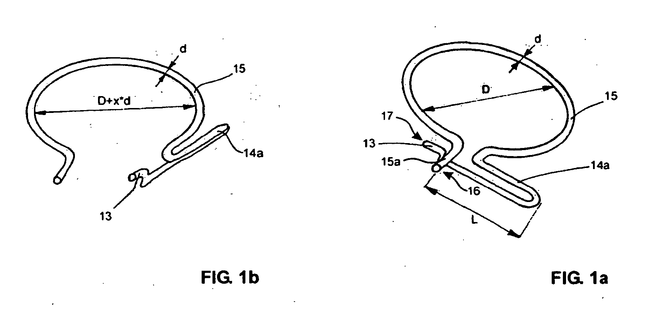

Wie oben ausgeführt, ist das Halteelement gemäß der Erfindung ein zu einem umlaufenden Ring verschließbares Element. Unter dem Begriff "ein zu einem umlaufenden Ring verschließbares Element" wird dabei nicht streng eine kreisrunde geometrische Struktur verstanden, sondern dies besagt, dass das Halteelement das Antriebsmittel umgibt. Dabei braucht es nicht streng das Antriebsmittel, beispielsweise die Kolbenstange, nachbilden, sondern es ist wesentlich, dass das Halteelement an möglichst vielen Punkten anliegt. Umfasst von dem Begriff sind deshalb beispielsweise ebenso elliptische oder polygonale Formen. Weiter kann der Ring auch im Verschlussbereich etwas geöffnet bleiben, d.h. dort nicht an dem Antriebsmittel anliegen, wie beispielsweise anhand der Fig. 1 deutlich wird.

Nachfolgend wird die Erfindung anhand der Zeichnungen näher erläutert und beschrieben. Es zeigen:
- Fig. 1:: perspektivische Darstellungen des erfindungsgemäßen Halteelementes im geschlossenen Zustand (Fig. 1a) und im geöffneten Zustand (Fig. 1b);
- Fig. 2:: ein Längsschnitt durch eine erfindungsgemäße Injektionsvorrichtung; und
- Fig. 3:: das Detail III aus Figur 2 in vergrößerter Darstellung, wobei die eine Halbansicht das Detail im Schnitt und die andere Halbansicht es in Seitenansicht zeigt.

In den Figuren ist als Beispiel einer erfindungsgemäßen Verabreichungsvorrichtung eine Injektionsvorrichtung gezeigt. Weiterhin ist als Beispiel eines erfindungsgemäßen Antriebsmittels eine Kolbenstange dargestellt und beschrieben.

In Fig. 1a ist das Halteelement 15 in geschlossenem Zustand, d.h. vor dem Auslösen gezeigt. Der Innendurchmesser D des Halteelementes 15 entspricht annähernd dem Durchmesser einer Kolbenstange einer erfindungsgemäßen Injektionsvorrichtung an der Stelle, wo das Halteelement 15 die Kolbenstange umschließt. Das Halteelement 15 hat im Wesentlichen eine Ringform, wobei der Ring ein geöffneter Ring ist. Zur Fixierung des Halteelementes 15 in einer geschlossenen Position ist an dem ersten Ende 16 des Halteelementes eine Einhaknase 15a vorgesehen, unter welche das zweite Ende 17 untergehakt ist. Zur Arretierung im geschlossenen Zustand ist das zweite Ende 17 zu einer Stufe 13 gebogen. Das Halteelement 15 ist im geschlossenen Zustand vorgespannt. Durch diese Vorspannung springt es nach Lösen der Verhakung von selbst in den geöffneten, d.h. entspannten Zustand, der in Figur 1b dargestellt ist.

Aus einem Vergleich der Figuren 1a und 1b ergibt sich, dass die ineinander verhakten Enden 16 und 17 zum Öffnen des Haltelementes voneinander getrennt werden müssen. Hierfür gibt es verschiedene Möglichkeiten. Entscheidend ist, dass durch das Auslösen die ineinander verhakten Enden 16 und 17 voneinander getrennt werden. So kann zum einen auf die Stufe 13 gedrückt werden, wodurch das erste Ende 16 frei wird und das Halteelement 15 aufspringen kann. Alternativ dazu kann das erste Ende 16 über die Stufe 13 hinweg gehoben werden, wodurch sich das Halteelement 15 ebenfalls entspannen und damit öffnen kann. Bei diesen beiden Fällen wirkt die Auslösekraft folglich in etwa senkrecht auf die durch das Halteelement aufgespannte Ebene ein. Schließlich ist auch durch eine Seitwärtsbewegung eine Öffnung des Elementes 15 möglich. Hierbei wirkt die Auslösekraft somit im Wesentlichen parallel zu der durch das Halteelement 15 aufgespannten Ebene. Es kann zum Beispiel die Stufe 13 seitlich unter dem ersten Ende 16 hervor gezogen werden. Die Bewegung, die zur Auslösung führt, kann sowohl translatorisch als auch rotatorisch sein. So ist z. B. neben einer radialen Bewegung auch eine Drehbewegung möglich, mit der die beiden Enden 16, 17 voneinander gelöst werden. Es wird ferner darauf hingewiesen, dass auch andere Ausführungsformen bedacht sind, die eine lösbare Verbindung der beiden Enden 16 und 17 ermöglichen, beispielsweise eine Haken-Ösen-Verbindung.

Das in den Figuren 1a und 1b dargestellte Halteelement 15 weist einen Kragen 14a auf. Bei diesem Kragen 14a handelt es sich um eine Schleife, die das zweite Ende 17 bildet, ehe es in die Stufe 13 übergeht. Das zweite Ende 17 geht folglich in dem bevorzugten Ausführungsbeispiel nicht unmittelbar in die Stufe 13 über sondern bildet eine Auskragung aus. Der Kragen 14a bildet eine Art Hebelarm. Wie oben ausgeführt, ist in einem bevorzugten Ausführungsbeispiel vorgesehen, dass das Lösen des Halteelementes 15 durch Drücken auf die Stufe 13 erfolgt. Mit Hilfe der Länge L des Kragens 14a ist die zum Auslösen nötige Kraft bestimmbar. Je länger der Kragen 14a ist, desto geringer ist die Auslösekraft.

Der Durchmesser d gibt den Durchmesser des Halteelementes 15 selber an, d.h. den Durchmesser des Materials, beispielsweise des Drahtes aus Federstahl, aus welchem das Halteelement 15 gebildet ist. Das Halteelement 15 ist so ausgelegt, dass der Innendurchmesser D nach dem Auslösen auf den Innendurchmesser D' = D + x * d erweitert wird, wodurch die Kolbenstange freigegeben wird. Ein bevorzugter Wert für x beträgt x ≥ 4. Der Durchmesser d des Haltelementes 15 ist bevorzugt d ≤ 0,1 * D, wobei D den Innendurchmesser des Halteelementes im geschlossenen Zustand bezeichnet.

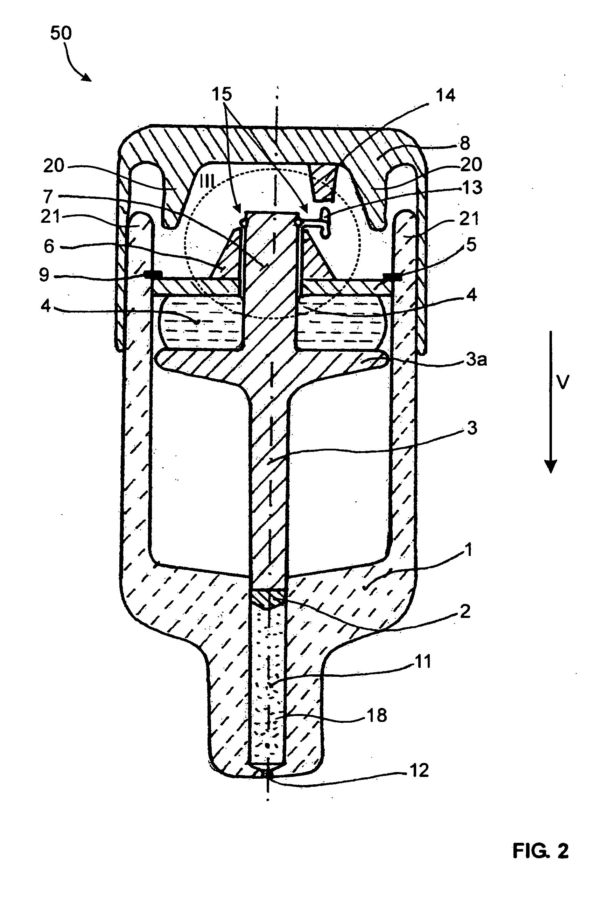

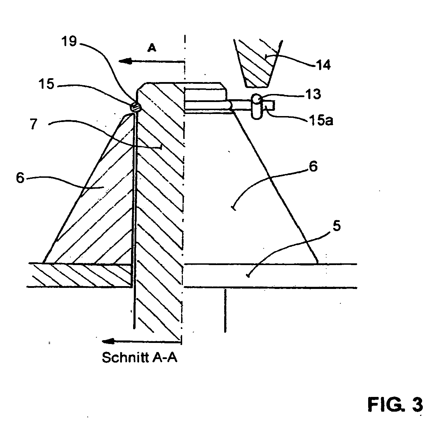

Die Figuren 2 und 3 zeigen als Ausführungsbeispiel einer erfindungsgemäßen Verabreichungsvorrichtung ein Injektionsgerät 50 im Längsschnitt (Fig. 2) bzw. in einer Detaildarstellung (Fig. 3). Bei dem Injektionsgerät 50 der Fig. 2 handelt es sich um ein Gerät zum einmaligen Gebrauch, das keine Nadel aufweist, sondern bei dem das zu injizierende Produkt 11 durch einen entsprechend großen Druck unter die Haut eines Patienten gespritzt wird. Das zu injizierende Produkt 11 befindet sich in einem Kanal 18, der zur Hautseite des Patienten hin, d.h. in distaler Richtung, von einer Öffnung 12 und in proximaler Richtung, d.h. von der Haut abgewandt, von einem Kolben 2 abgeschlossen wird. Der Kolben 2 ist mit einer Kolbenstange 3 gekoppelt, so dass der Kolben 2 durch die Kolbenstange 3 in Vorschubrichtung V bewegt werden kann. Die Kolbenstange 3 weist einen Kolbenstempel 3a auf, d.h. die Kolbenstange 3 ist durch einen Vorsprung erweitert. Zwischen dem Kolbenstempel 3a und einem gehäusefesten Flansch 5 des Injektionsgerätes ist ein Druckmittel 4 in Halteposition, wie es in Figur 2 dargestellt ist, gespannt. Als Druckmittel 4 ist beispielsweise eine Torsionsfeder oder eine entropische Feder vorgesehen. Die Kolbenstange 3 ist über den Flansch 5 hinaus verlängert, wo sie einen Schaft 7 bildet.

Der Schaft 7 und damit die Kolbenstange 3 wird durch das Halteelement 15 im Halteeingriff lösbar gehalten, wodurch die Kolbenstange 3 in Halteposition fixiert wird. Das Halteelement 15 sitzt im Ausführungsbeispiel der Fig. 2 und 3 auf einem Haltesitz 6 auf, bei dem es sich um ein mit dem Flansch 5 verbundenes bzw. an den Flansch 5 angeformtes keilförmiges oder kegelförmiges Element handelt. Der Haltesitz 6, der mit dem Halteelement 15 zusammenwirkt, kann aus Kunststoff gefertigt sein. Wie oben ausgeführt, verteilt sich bedingt durch die Ringauflagefläche des Halteelementes 15 der Auflagedruck über die gesamte Ringfläche. Bevorzugt bestehen der Flansch 5 und der Haltesitz 6 aus Kompositfaser, beispielsweise sind sie durch Spritzguss einstückig hergestellt. Während der Lagerung des Injektionsgerätes 50, während der das Druckmittel 4 gespannt ist, verteilt sich die Last auf die Kolbenstange 3, den Haltesitz 6 und den Flansch 5.

Prinzipiell reicht die Klemm- und Keilwirkung des Halteelementes 15 aus, die Kolbenstange 3 in Halteposition zu halten. Allein dadurch wird eine sichere Fixierung erzielt. Für einen besonders sicheren Halt ist jedoch bevorzugt am Schaft 7 der Kolbenstange 3 eine Nute 19 vorgesehen. Alternativ dazu kann auch am proximalen Ende des Schaftes 7 ein entsprechender Vorsprung bzw. Wulst ausgebildet sein, unter dem eine Verankerung erfolgt.

Am proximalen Ende wird das Injektionsgerät 50 von einer Auslösekappe 8 abgeschlossen. Zum Auslösen des Injektionsgerätes 50 wird die Auslösekappe 8 in Vortriebsrichtung V bewegt, d.h. es wird auf die Auslösekappe 8 gedrückt. Dadurch wird ein an der Auslösekappe 8 befestigter oder angeformter Vorsprung 14 nach unten bewegt und drückt auf die Stufe 13 des Halteelementes 15. Der Vorsprung 14 kann verschiedene Formen annehmen, z. B. Stift- oder Zapfenform. Wie bereits zu den Figuren 1 a und 1 b beschrieben, wird durch das Einwirken des Vorsprungs 14 das Halteelement 15 gelöst, es springt auf und gibt die Kolbenstange 3 frei. Der Ringvorsprung 20, der an der Auslösekappe 8 ausgebildet ist, stellt in Verbindung mit den Dämpferelementen 9 einen Anschlag dar, so dass die Auslösekappe 8 nur über eine definierte Weglänge gedrückt werden kann. Ferner stellt der Ringvorsprung 20 in Verbindung mit dem Gehäuse 1 des Injektionsgerätes 50 eine Führung bereit. Die Schulter 21 stellt ebenfalls eine Anschlagsbegrenzung bereit.

Anstelle einer Auslösekappe 8, wie in den Fig. 2 und 3 sind auch andere Formen an Auslösern bedacht. So ist beispielsweise ein Auslöser vorgesehen, der für den Auslösevorgang hochgezogen werden muss. Weiter kann der Auslöser auch so ausgebildet sein, dass er durch ein Drücken von der Seite her betätigbar ist.

Das erfindungsgemäße Injektionsgerät 50 funktioniert wie folgt: Der Patient setzt das Injektionsgerät 50 mit der Öffnung 12 auf die Haut an der Stelle auf, wo das Produkt 11 injiziert werden soll. Er drückt nun auf die Auslösekappe 8, wodurch diese entlang dem Gehäuse 1 des Injektionsgerätes 50 gleitet und mit Hilfe des Vorsprunges 14 auf die Stufe 13 des Halteelementes 15 drückt. Wurde der Vorsprung 13 ausreichend weit gedrückt, so springt das Halteelement 15 auf, wodurch die Kolbenstange 3 freigegeben wird. Durch die Freigabe der Kolbenstange 3 entlädt sich die Kraft des Druckmittels 4, das in Halteposition gespannt vorliegt. Im Falle einer entropischen Feder wird eine nichtlineare Kraft auf den Druckstempel 3a ausgeübt, wodurch der Kolben 2 in Vortriebsrichtung bewegt und damit das Produkt 11 injiziert wird. Bei Verwendung der entropischen Feder wird zunächst ein sehr hoher Druck erzeugt, wodurch die Penetration der Haut erfolgt. Im weiteren Verlauf wird dagegen der Druck reduziert, so dass das Produkt 11 langsam injiziert wird.

## Patentansprüche

1. Verabreichungsvorrichtung (50), umfassend
- ein Gehäuse (1), in dem ein zu applizierendes Produkt (11) aufgenommen ist,
- einen Kolben (2), der in dem Gehäuse in Vortriebsrichtung (V) bewegbar aufgenommen ist, um das Produkt (11) auszuschütten, und
- ein in Vortriebsrichtung (V) auf ein Antriebsmittel (3) wirkendes Druckmittel (4),
wobei das Antriebsmittel (3) mit Hilfe eines Halteelementes (15) gegen die Kraft des Druckmittels (4) in einer Halteposition in einem Halteeingriff lösbar gehalten wird,
**dadurch gekennzeichnet, dass**
das Halteelement (15) ein zu einem umlaufenden Ring verschließbares Element ist, das im geschlossenen Zustand das Antriebsmittel (3) in Halteeingriff hält und vorgespannt ist und das im geöffneten Zustand das Antriebsmittel (3) freigibt.

2. Verabreichungsvorrichtung nach Anspruch 1, **dadurch gekennzeichnet, dass** das Halteelement (15) an seinem ersten Ende (16) eine Einhaknase (15a) aufweist, in die das zweite Ende (17) im geschlossenen Zustand eingehakt ist, wobei das zweite Ende bevorzugt eine Stufe (13) aufweist.

3. Verabreichungsvorrichtung nach Anspruch 2, **dadurch gekennzeichnet, dass** das zweite Ende (17) einen Kragen (14a) aufweist, wobei über die Länge (L) des Kragens (14a) die zum Auslösen notwendige Kraft bestimmbar ist.

4. Verabreichungsvorrichtung nach einem der vorstehenden Ansprüche, **dadurch gekennzeichnet, dass** ein Haltesitz (6) vorgesehen ist, der mit dem Halteelement (15) zusammenwirkt, wodurch das Antriebsmittel (3) in Halteposition in dem Halteeingriff lösbar gehalten wird.

5. Verabreichungsvorrichtung nach einem der vorstehenden Ansprüche, **dadurch gekennzeichnet, dass** die Auslösekraft senkrecht oder parallel zu einer durch das Halteelement (15) aufgespannten Ebene wirkt.

6. Verabreichungsvorrichtung nach einem der vorstehenden Ansprüche, **dadurch gekennzeichnet, dass** die Verabreichungsvorrichtung einen Auslöser, insbesondere eine Auslösekappe (8) aufweist, an der ein Vorsprung (14) angebracht ist, der bei Betätigen des Auslösers auf eines der beiden Enden (16, 17) einwirkt und so die Öffnung des Halteelementes (15) bewirkt.

7. Verabreichungsvorrichtung nach einem der vorstehenden Ansprüche, **dadurch gekennzeichnet, dass** an dem Antriebsmittel (3) eine Nute (19) oder ein Vorsprung vorgesehen ist, in der bzw. unter dem das Halteelement (15) im geschlossenen Zustand gehalten wird.

8. Verabreichungsvorrichtung nach einem der vorstehenden Ansprüche, **dadurch gekennzeichnet, dass** das Halteelement (15) im geöffneten Zustand einen Innendurchmesser D' aufweist, wobei D' = D + x * d ist, wobei D der Innendurchmesser des Halteelementes (15) im geschlossenen Zustand, d der Durchmesser des Halteelementes (15) selber und x ≥ 4 ist.

9. Verabreichungsvorrichtung nach einem der vorstehenden Ansprüche, **dadurch gekennzeichnet, dass** das Druckmittel (4) eine Torsionsfeder oder eine entropische Feder ist.

10. Verabreichungsvorrichtung nach einem der vorstehenden Ansprüche, **dadurch gekennzeichnet, dass** das Halteelement (15) aus Federstahl oder einem Kompositwerkstoff gefertigt ist, oder dass es mit Faserverbundstoffen armiert ist.

## Claims

1. An administration device (50) comprising
- a housing (1) in which a product (11) to be applied is accommodated,
- a piston (2) which is accommodated in the housing movably in the advance direction (V) in order to expel the product (11), and
- a pressure means (4) acting on a drive means (3) in the advance direction (V),
wherein the drive means (3) is releasably held in a holding position in a holding engagement against the force of the pressure means (4) by means of a holding element (15),
**characterised in that**
the holding element (15) is an element which is closable to afford a peripherally extending ring and which in the closed condition holds the drive means (3) in holding engagement and is prestressed and which in the opened condition releases the drive means (3).

2. An administration device according to claim 1 **characterised in that** at its first end (16) the holding element (15) has a hooking engagement nose (15a) into which the second end (17) is hookingly engaged in the closed condition, wherein the second end preferably has a step (13).

3. An administration device according to claim 2 **characterised in that** the second end (17) has a collar (14a), wherein the force necessary for triggering can be determined by way of the length (L) of the collar (14a).

4. An administration device according to one of the preceding claims **characterised in that** there is provided a holding seat (6) co-operating with the holding element (15) whereby the drive means (3) is releasably held in a holding position in the holding engagement.

5. An administration device according to one of the preceding claims **characterised in that** the triggering force acts perpendicularly or parallel to a plane defined by the holding element (15).

6. An administration device according to one of the preceding claims **characterised in that** the administration device has a triggering means, in particular a triggering cap (8) on which there is provided a projection (14) which upon actuation of the triggering means acts on one of the two ends (16, 17) and thus causes opening of the holding element (15).

7. An administration device according to one of the preceding claims **characterised in that** provided on the drive means (3) is a groove (19) or a projection in which or under which the holding element (15) is held in the closed condition.

8. An administration device according to one of the preceding claims **characterised in that** in the opened condition the holding element (15) has an inside diameter D', wherein D' = D + x * d, wherein D is the inside diameter of the holding element (15) in the closed condition, d is the diameter of the holding element (15) itself and x ≥ 4.

9. An administration device according to one of the preceding claims **characterised in that** the pressure means (4) is a torsion spring or an entropic spring.

10. An administration device according to one of the preceding claims **characterised in that** the holding element (15) is made from spring steel or a composite material or is reinforced with fibre composites.

## Revendications

1. Dispositif d'administration (50), comprenant :
- un boîtier (1) dans lequel est reçu le produit à appliquer (11),
- un piston (2) qui est reçu dans le boîtier en étant déplaçable dans une direction d'avance (V) pour chasser le produit (11), et
- un milieu sous pression (4) agissant sur un moyen d'entraînement (3) en direction d'avance (V),
le moyen d'entraînement (3) étant maintenu de façon libérable à l'aide d'un élément de maintien (15) à l'encontre de la force du milieu sous pression (4) dans une position de maintien dans un engagement de maintien,
**caractérisé en ce que**
l'élément de maintien (15) est un élément capable d'être refermé pour former un anneau périphérique qui, dans l'état fermé, maintient le moyen d'entraînement (3) en engagement de maintien et est précontraint, et qui, dans l'état ouvert, libère le moyen d'entraînement (3).

2. Dispositif d'administration selon la revendication 1, **caractérisé en ce que** l'élément de maintien (15) présente à sa première extrémité (16) un ergot d'accrochage (15a) dans lequel la seconde extrémité (17) est accrochée dans l'état fermé, ladite seconde extrémité présentant de préférence un gradin (13).

3. Dispositif d'administration selon la revendication 2, **caractérisé en ce que** la seconde extrémité (17) présente une collerette (14a), la force nécessaire pour le déclenchement étant déterminable via la longueur (L) de la collerette (14a).

4. Dispositif d'administration selon l'une des revendications précédentes, **caractérisé en ce qu'**il est prévu un siège de maintien (6) qui coopère avec l'élément de maintien (15), ce grâce à quoi le moyen d'entraînement (3) est maintenu de façon libérable en position de maintien dans l'engagement de maintien.

5. Dispositif d'administration selon l'une des revendications précédentes, **caractérisé en ce que** la force de déclenchement agit perpendiculairement ou parallèlement à un plan défini par l'élément de maintien (15).

6. Dispositif d'administration selon l'une des revendications précédentes, **caractérisé en ce que** le dispositif d'administration comprend un déclencheur, en particulier un capuchon déclencheur (8), sur lequel est ménagée une saillie (14) qui, lors de l'actionnement du déclencheur, agit sur l'une des deux extrémités (16, 17) et provoque ainsi l'ouverture de l'élément de maintien (15).

7. Dispositif d'administration selon l'une des revendications précédentes, **caractérisé en ce que** sur le moyen d'entraînement (3) il est prévu une rainure (19) ou une saillie dans laquelle, ou respectivement sous laquelle, l'élément de maintien (15) est maintenu dans l'état fermé.

8. Dispositif d'administration selon l'une des revendications précédentes, **caractérisé en ce que** l'élément de maintien (15) présente à l'état fermé un diamètre intérieur D', tel que D' = D + x * d, D étant le diamètre intérieur de l'élément de maintien (15) à l'état fermé, d étant le diamètre de l'élément de maintien (15) lui-même, et x ≥4.

9. Dispositif d'administration selon l'une des revendications précédentes, **caractérisé en ce que** le moyen sous pression (4) est un ressort de torsion ou un ressort entropique.

10. Dispositif d'administration selon l'une des revendications précédentes, **caractérisé en ce que** l'élément de maintien est réalisé en acier à ressort ou en un matériau composite, ou **en ce qu'**il est armé avec des matériaux composites à fibres.
